# EUROPEAN PATENT SPECIFICATION

(11) **EP 1 385 480 B1**
(45) Date of publication and mention of the grant of the patent: **06.05.2009**
(21) Application number: 02769160.9
(22) Date of filing: 10.05.2002
(51) Int. Cl.: A61K 9/127

(54) **LIPOSOMES**
LIPOSOMEN
LIPOSOMES

(30) Priority: 10.05.2001 GB 0111279
(43) Date of publication of application: 04.02.2004
(73) Proprietor: GE HEALTHCARE AS, 0485 Oslo (NO)
(72) Inventor: FOSSHEIM, Sigrid, Amersham Health AS, N-0401 Nydalen (NO); ODEGARDSTUEN, Liv, Ingrid, Amersham Health AS, N-0401 Nydalen (NO)
(74) Representative: Rollins, Anthony John
(86) International application number: PCT/GB2002/002100
(87) International publication number: WO 2002/089771

(56) References cited:
- EP-A- 0 170 247
- WO-A-00/66126
- WO-A-94/13265
- US-A- 5 756 121

## Description

### Field of the invention

This invention relates to liposomes of a specific bilayer composition containing an entrapped modifying compound in their internal phase, said modifying compound being capable of chemically modifying subsequently internalised material, such that said material remains internalised within the liposomes. A variety of materials are suitable for internalisation, however the liposomes of the invention are particularly suitable for internalisation of metal chelates. The liposomes are useful for imaging infection, inflammation or tumour in humans and for the effective delivery of drug substances.

### Background of the invention

Liposomes are vesicles consisting of a phospholipid bilayer enclosing an aqueous interior. Encapsulation of material in the aqueous interior enables the accumulation of that material in target tissues and decreases its spread to nontarget tissues where it might do harm. This is an especially useful mechanism where the material is a drug with toxic side effects. Liposomes are a popular delivery system for such drugs; e.g., the cancer therapeutic epipodophyllotoxin produces leucopenia, thrombocytopenia and anemia if administered systemically. WO 00/09071 1 discloses a liposomal preparation of epipodophyllotoxin as a means to avoid these systemic side effects. Similarly, WO 96/17596 discloses a liposomal composition comprising a-interferon, and WO 92/02208 discloses a liposomal composition comprising doxorubicin.

Liposomes are also of considerable interest because of their value as carriers for diagnostic agents. Examples are diagnostic agents for magnetic resonance imaging (MRI), single photon emission tomography (SPECT), ultrasound and x-ray. Radiopharmaceuticals for tracer and SPECT imaging studies have been of particular interest. Various means have been evaluated and detailed accounts of radiolabelling liposomes may be found in the scientific literature.

-The-most common radiolabel used in diagnostic nuclear medicine today is ^{99m}Tc. This radiolabel is produced from the beta decay of molybdenum-99 (⁹⁹Mo) and has a half-life of 6 hours. It is widely available from a generator system at low cost, and its relatively short half-life provides for safer and more convenient handling than other available radiolabels. Its gamma emission is 141keV, which is ideal for producing high-resolution images. ⁹⁹TcO₄⁻ is produced from a generator and since it is relatively unreactive, must be reduced to a lower oxidation state before use as a radiopharmaceutical. Stannous chloride is the most commonly used reducing agent. Incubation of liposomes with ^{99m}TcO₄⁻ solution and stannous chloride has been evaluated as a means to radiolabel liposomes. The radiolabelling efficiency of that method was not reproducible, and removal of excess reducing agent, free pertechnetate and ^{99m}Tc-SnCl₂ colloid were problems (Barratt, G.M., Tüzel, N.S., and Ryman, B.E., Liposome Technology Volume II, Gregoridas, G., Ed, CRC Press, Boca Raton, (1984)). Also in this document, ^{99m}Tc was reported to be released from liposomes both *in vitro* and *in vivo.* These issues resulted in liposomes of limited usefulness for many applications.

Octadecylamine-DTPA incorporated in liposomes has been shown to rapidly and efficiently label liposomes with ⁶⁷Ga or ^{99m}Tc by chelation, but over 30% of the label was lost after a 2 hour incubation in plasma.

Multilamellar lipid vesicles labelled with ¹¹¹In using 8-hydroxyquinoline showed a labelling efficiency of 30% (Caride, V.J. and Sostman, H.D. in Liposome Technology Volume II, Gregoridas, G., Ed, CRC Press, Boca Raton, (1984)). Acetylacetone, a water soluble lipophilic chelator, can be complexed with ¹¹¹In. This is then mixed with liposome-encapsulated nitriloacetic acid with subsequent formation of labelled nitriloacetic acid. The resulting labelled liposomes are unstable unless excess acetylacetone is removed by an ion exchange process (Beaumier, P.L. and Hwang, K.J., J. Nuc. Med., 23, 810-815 (1982)).

Another method of preparing ^{99m}Tc-Iabelled liposomes involves the incubation of liposomes with a radiolabel in the form of a complex that acts as a carrier for the radiolabel. Such a method was disclosed in the early 1990s (Phillips, W.T. et al, Nuc. Med. Biol., 19, 539-547 (1992); US 5143713). The preferred carrier is hexamethylenepropylene amine oxime (HMPAO), which is labelled with ^{99m}Tc and incubated with liposomes at room temperature in order to produce radiolabelled liposomes. The ^{99m}TcHMPAO complex is lipophilic and is thus thought to enter the liposomes by passive diffusion across the lipid bilayer. ^{99m}Tc-HMPAO is thought to readily cross the membranes of negatively charged liposomes. The negatively charged liposomes of US 5143713 have a lipid composition in a mole ratio of neutral phospholipid:cholesterol:negatively charged phospholipid:α-tocopherol of 10:9:1 (Example 1, *sic).* Antioxidant entrapped within the liposomes is thought to convert lipophilic ^{99m}Tc-HMPAO to a hydrophilic form that remains in the internal aqueous phase of the liposome. However, the liposomes are only efficiently radiolabelled if the antioxidant remains chemically stable and entrapped within the liposomes.

Studies have revealed limited shelf life of these liposome suspensions due to the chemical lability of the reductant glutathione (Goins et al, J. Liposome Res., 8, 265, 1998).

The clinical uses of radiolabelled liposomes are well known in the art. Several applications in nuclear medicine have been proposed for liposomes including tumour imaging, infection and inflammation imaging and blood pool imaging.

Early biological studies used large multilamellar liposomes, which were predominantly cleared from the blood pool into the mononuclear phagocytic cells of the liver and spleen (Morgan, J.R. et al, J. Med. Microbiol., 14, 213-217 (1981)). The labelling procedure was also shown to be unstable, resulting in excessive loss of label from the liposomes. This instability was clearly seen in images of infected rats with high urinary excretion of free ^{99m}Tc. More recent work has used liposomes that have been processed to a size that minimises uptake into the mononuclear phagocyte system thus extending the blood circulation time. A study in rats infected with *S*. *aureus* demonstrated that abscesses were easily visualised within 2 hours of ⁹⁹Tc-liposome injection with a target to background ratio of 2.9 ± 0.3; increasing to 6.9 ± 1.1 by 24 hours (Goins, B. et al J. Nuc. Med., 34, 2160-2168 (1993)). The liposomes were labelled using the HMPAO carrier method, the labelling procedure appeared to be stable *in vivo* due to the low urinary excretion of ^{99m}Tc over a 24-hour period. The liposomes used comprised various lipids such as distearoylphosphatidylcholine (DSPC), dimyristoylphosphatidylglycerol (DMPG) and cholesterol. Both negatively charged DSPC:cholesterol:DMPG:α-tocopherol (50:38:10:2 molar ratio) and neutral DSPC:cholesterol:α-tocopherol (66:32:2 molar ratio) liposomes were studied. The liposomes were highly unilamellar and had a mean diameter of around 155nm. The tumours could be distinguished from the background muscle activity by 4 hours post-injection. The study also found that the neutral liposomes had increased uptake into the tumour and decreased nonspecific uptake by the mononuclear phagocyte system in comparison with the negatively charged liposomes.

For phospholipid vesicles that are in gel-phase at physiological temperature and below, the inclusion of cholesterol is known to fluidise the membranes. On the one hand, such fluidisation is desirable for the diffusion of material across the lipid bilayer. Conversely, it is not desirable in the context of retaining entrapped compounds within preformed liposomes. An additional consideration in determining optimal cholesterol content is the effect on biodistribution. It was recognised some years ago that prolonged circulation time would broaden the clinical applications of radiolabelled liposomes, e.g., tumour and inflammation/infection imaging. The cholesterol content has been shown to be a factor influencing the biodistribution and blood clearance kinetics; the fluidising effect of cholesterol renders the liposome membrane surface less susceptible to protein adsorption (i.e., reduced extent of opsonisation). For example, unilamellar cholesterol-rich liposomes of around 185nm diameter were tested in rats with focal *S. aureus* infection (Goins, B., et al, J. Nuc. Med., 34, 2160-2168 (1993)). The liposomes contained lipids in the molar ratio DSPC:cholesterol:DMPG:α-tocopherol, 50:38:10:2. These liposomes showed a circulatory half life of 10 hours in rats, which was considerably longer than that found for earlier formulations. Abscess to muscle ratios of up to 35 were documented with this formulation. In another study, it was found that liposomes containing DSPC and 20 mole percent cholesterol had a circulation half-life of 30 minutes in mice, as opposed to 5 hours for liposomes with DSPC and 30 mole percent cholesterol (Semple, S.C. et al Biochemistry, 35, 2521- (1996)). Based on the teachings of the prior art, the choice of sterol content ensuring both preservation of preferred physicochemical characteristics as well as *in vivo* biodistribution/pharmacokinetics is far from clear.

Preformed liposomes that are labelled by the end-user must be capable of long-term storage such that the physicochemical properties of the liposomes required for labelling and subsequent use *in vivo* are retained.

Lyophilisation in the presence of simple sugars is one potentially valuable means to prepare liposomes of a form suitable for long-term storage. Studies have shown that liposomes can be lyophilised in the presence of external sucrose and subsequently rehydrated with no change in their size (Tilcock, C. et al, Biochim. Biophys. Acta, 1148, 77-84 (1993)). Another study analysed the optimal concentration of sucrose for lyophilisation (Szucs, M. and Tilcock, C., Nucl. Med. Biol., 22, 263-268 (1995)). It was found that with 300mM sucrose the average diameter and size distribution were not significantly different before lyophilisation and after rehydration. The liposomes of these studies were surface labelled with ⁹⁹Tc following rehydration, a method which was found to result in leakage of ^{99m}Tc *in vivo*.

There is therefore a need for liposomes which (1) retain internalised material in its original chemical form over long-term storage, (2) retain their initial size over long-term storage, (3) can be loaded with an internalisable material, even following long-term storage and (4) may be used for the diagnostic imaging of infection, inflammation and tumours, or for the effective delivery of drug substances. It is an object of this invention to provide such liposomes. The most crucial physicochemical properties to consider for the liposome of the invention are liposome size, retention of entrapped material, and stability of liposome contents. Furthermore, these liposomes are also suitable for internalisation of certain MRI contrast agents, as well as prodrugs that are chemically converted to useful therapeutic compounds once inside the liposomes.

### Description of the invention

In a first aspect, the present invention provides novel liposomes which can be loaded with an intemalisable material to provide liposomes suitable for imaging infection, inflammation and tumour as well as for drug delivery. The composition results in liposomes that are physicochemically robust and that retain entrapped material in its original chemical form both during processing, e.g., dehydration, and during extended storage.

Liposomes of the present invention comprise:
a neutral phospholipid,
a negatively charged phospholipid,
a sterol,
having a modifying compound entrapped in the internal phase of said liposome;
wherein the modifying compound is a compound that is capable of irreversibly chemically modifying an intemalisable material within said liposome following diffusion of said material across the phospholipid bilayer of said liposome, such that the irreversibly chemically modified material remains internalised within said liposome, and;
wherein the neutral phospholipid is present at 60-90 mole percent of the total lipid content, the negatively charged phospholipid is present at 2-20 mole percent of the total lipid content and the sterol is present at 5-25 mole percent of the total lipid content.

A "modifying compound" is defined in the present invention as a compound that is capable of irreversibly chemically modifying material that has been internalised within preformed liposomes such that said material remains internalised within the liposome. The modifying compound is entrapped within the liposomes during their manufacture and ideally remains substantially entrapped, as well as chemically unchanged, during preparation for extended storage and over extended storage periods. Following such extended storage, the liposomes can suitably be used to internalise material by diffusion of said material across the phospholipid bilayer, followed by chemical modification of the material within the liposomes. The chemical modification induced by the modifying compound is suitably a modification that is irreversible and which prevents the internalised material from diffusing out of the liposomes. Modifications outside the scope of the present invention are reversible chemical reactions e.g. protonation reactions, as used in pH-mediated remote drug loading into liposomes.

Suitable modifying compounds of the present invention include chemical reactants such as oxidants, reductants or transchelators. Preferable reductants of the invention are the antioxidants ascorbic acid, glutathione and cysteine. A most preferred antioxidant modifying compound is glutathione. The modifying compound remains entrapped within the liposomes of the invention over extended storage periods in its original chemical form. The efficient transmembrane passage and loading of the intemalisable material into said liposomes following extended storage of the liposomes is thus permitted. The contradictory requirements of retaining entrapped modifying compound both during the preparation for long-term storage and during long-term storage, while allowing subsequent transmembrane passage and internalisation of material are fulfilled using the lipid bilayer compositions of the present invention.

The stability of the modifying compound may be pH-dependent. Thus, a further feature of the present invention is an adjustment of the composition of the intraliposomal and extraliposomal aqueous phases. For example, when the modifying compound is glutathione, the intraliposomal pH is preferably between 3 and 6 to minimise oxidation and hydrolysis of the glutathione. The preferred intraliposomal pH may be achieved with the use of buffering systems. The preferred internal phase is phosphate- or citrate-phosphate buffered sucrose solutions. The preferred external phase for successful preservation of physicochemical properties during preparation for long-term storage is a buffer-free sucrose solution.

Suitable material for internalisation into the liposomes of the invention can be a radioactive SPECT imaging agent, an MRI contrast agent, an x-ray contrast agent or a prodrug. A suitable prodrug is one which can passively diffuse across the lipid bilayer of the liposome of the invention, and it is suitably converted into the corresponding drug upon intemalisation. Said drug suitably does not diffuse across the liposome membrane. Preferred materials for internalisation are radioactive compounds, paramagnetic compounds and radioopaque compounds: The internalisation of such materials produces liposomes that are suitable for the effective imaging of infection, inflammation and tumour in humans. Most preferred materials of the invention are metal chelates, wherein the metal of said metal chelates is suitably chosen from a radiometal, a paramagnetic metal or a radioopaque metal. Preferred radiometals are ^{99m}Tc and ¹¹¹In, suitable paramagnetic metals are gadolinium and manganese and a suitable radioopaque metal is tungsten. A most preferred radiometal is ^{99m}Tc, wherein the preferred metal chelate is ^{99m}Tc-HMPAO.

The "total lipid content" is defined in the present invention as being the mole sum of all the lipid components present in the lipid bilayer of the liposomes. These lipid components are the various phospholipids plus the sterol. The amount of each lipid component present in the lipid bilayer of the liposomes is expressed in terms of mole percent of the total lipid content, i.e., the total lipid content thus corresponds to 100 mole percent.

The neutral phospholipid is preferably present between 70 and 85 mole percent of the total lipid content. The negatively charged phospholipid is preferably present between 5 and 10 mole percent of the total lipid content. A preferred embodiment is liposomes with a bilayer composition wherein the sterol is present between 10 and 25 mole percent of the total lipid content.

The sterol component in the liposomes of the present invention is suitably cholesterol or its derivatives, e.g., ergosterol or cholesterolhemisuccinate, but is preferably cholesterol. The sterol is present in an amount that both (1) enables maximum retention of entrapped modifying compound, and (2) minimises alterations in physicochemical properties (e.g., liposome size and size distribution), during preparation for long-term storage while, (3) permitting the passage of material into the internal phase of the liposome, even after extended storage periods. Example 1 shows that DSPC:DMPG:cholesterol (mole ratio; between 49:5:7.5 and 49:5:15) liposomes generally demonstrated a reduced extent of glutathione (oxidised and reduced form) leakage, a marginal change in the liposome size and a smaller change in radiochemical purity (RCP) post-lyophilisation, compared to DSPC:DMPG:cholesterol (mole ratio; 49:5:44) liposomes (Table I). The inclusion of a sterol such as cholesterol into the phospholipid bilayer is known to influence membrane fluidity. The present invention provides liposomes of an optimised sterol content which are sufficiently stable *in vitro* for extended shelf-life, maintain their desirable physicochemical characteristics throughout and, following internalisation of material have a biodistribution profile suitable for *in vivo* use. Liposome size was also demonstrated to affect the pharmacokinetics and biodistribution, as shown in Example 6. Similar percentage blood radioactivity and lesion uptake was found for similar-sized lyophilised DSPC:DSPG:cholesterol and DSPC:DMPG:cholesterol liposomes (mole ratio between 49:5:7.5 and 49:5:20) and liposome suspensions with high cholesterol content (DSPC:DMPG:cholesterol:a-tocopherol; mole ratio; 49:5:44:2).

The lipid bilayer of the liposomes of the present invention preferably contains negatively charged and neutral phospholipid components that have fatty acid portions with acyl chains of between 14 and 20 carbon atoms. The neutral phospholipid component of the lipid bilayer is preferably a phosphatidylcholine, most preferably chosen from distearoylphosphatidylcholine (DSPC) and dimyristoylphosphatidylcholine (DMPC). The negatively charged phospholipid component of the lipid bilayer may be a phosphatidylglycerol, phosphatidylserine or phosphatidylinositol compound, preferably chosen from distearoylphosphatidylglycerol (DSPG) and dimyristoylphosphatidylglycerol (DMPG). Preferably, the neutral phospholipid is a phosphatidylcholine compound and the negatively charged phospholipid is a phosphatidylglycerol compound. The fatty acid portion of these phospholipids may be chosen from myristic acid, palmitic acid, stearic acid, oleic acid, linoleic acid or archidonic acid. Preferred fatty acid portions are myristic acid and stearic acid, with fatty acid portions with acyl chain lengths of 14 and 18 carbons, respectively. It is a most preferred embodiment of this invention that the fatty acid portions of both the negatively charged and neutral phospholipid components are of equal acyl chain length.

The liposomes of the present invention do not require the use of α-tocopherol as a component of their membranes. α-tocopherol was included in some prior art radiolabelled liposomes as an antioxidant necessary to limit oxidation of the phospholipids as well as any entrapped components, and thus ensure acceptable RCP. The inclusion of α-tocopherol was found not to be an essential requirement for the liposomes of the present invention, and acceptable RCP values were achieved with the absence of α-tocopherol in the lipid composition.

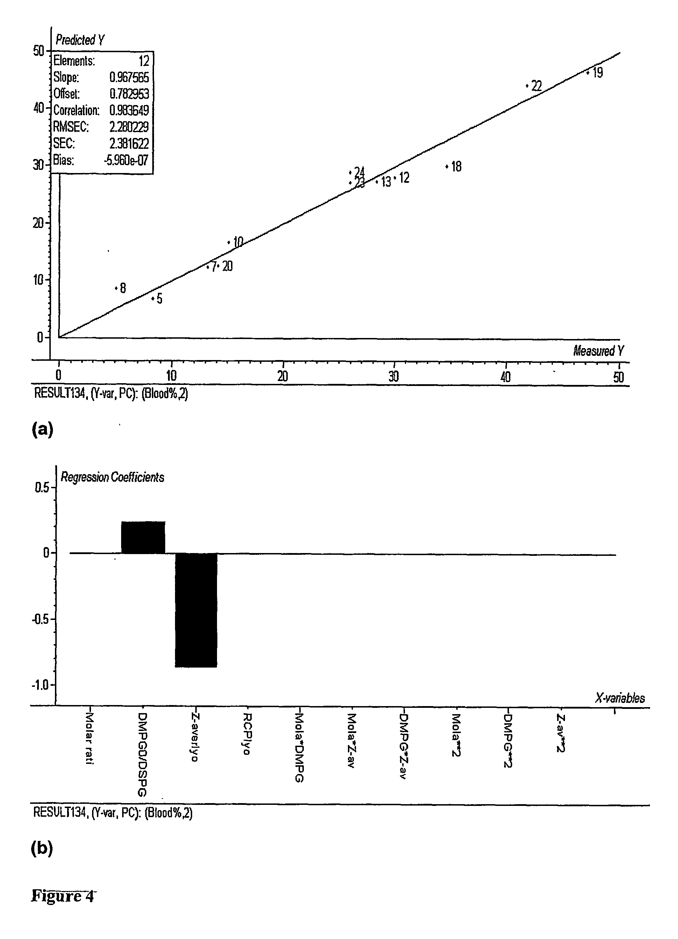

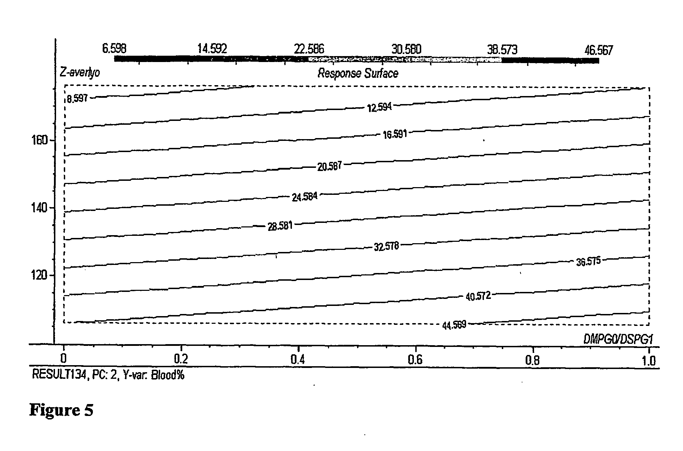

The blood half-life of the liposomes *in vivo* must be sufficiently long, and therefore the liposomes of the invention must be of a suitable size. Liposomes of the present invention are suitably of a diameter between 50 and 400nm and preferably of a diameter between 80 and 140nm. Example 6 shows that a decrease in liposome size increased the percentage radioactivity in the blood, a measurement that is indicative of blood residence time. It was also shown that the use of DSPG rather than DMPG as the negatively charged phospholipid increased slightly the percentage blood radioactivity for a given liposome size. For liposomes with the diameter range 130-180 nm, where size and lesion uptake were inversely correlated, the extent of lesion uptake was similar for both lyophilised DSPG- and DMPG-based liposomes. These conclusions were supported by a combined multivariate evaluation of the biodistribution data for both lyophilised DSPG- and DMPG based liposome preparations. Figure 4 depicts a model predicting percentage blood radioactivity (4 hours post injection), with the liposome size being the primary modulator. Figure 5 shows the predicted percentage blood radioactivity (4 hours post injection) as a function of liposome size, DMPG and DSPG.

Liposomes of the present invention were prepared by methods that are broadly known in the art (Lasic DD. Preparation of liposomes. In: Lasic DD, ed. Liposomes from physics to applications. Amsterdam, The Netherlands: Elsevier Science Publishers B.V., 1993; 63-107). The method of preparation is described briefly in Example 1.

In a second aspect of the present invention a liposome having an internalised material is provided which comprises the liposome of the first aspect of the invention and wherein the internalised material has been irreversibly chemically modified by the modifying compound. The preparation of liposomes having internalised material typically takes place concurrently with reconstitution of the liposomes following extended storage. Preferably, the internalised material is either a SPECT imaging agent, an MRI contrast agent, an x-ray contrast agent or a drug. Most preferably the internalised material is a radioactive compound, a paramagnetic compound, or a radioopaque compound. Especially preferred internalised material is a radiometal. A radiometal is preferably complexed with a chelating agent suitable for transportation of said radiometal into the liposomes. A suitable chelating agent must be capable of complexing with the desired radiometal and diffusing through the liposomal membrane. This will require a carrier that forms a radiometal complex that is lipophilic and sufficiently water soluble to permit efficient transfer within the water compartment of the lipid vesicle.

Where the internalised material is a SPECT imaging agent, it is preferably a gamma emitter, with ^{99m}Tc and ¹¹¹In being particularly useful in imaging of human subjects. A most preferred gamma emitter of the invention is ^{99m}Tc. A suitable chelate for ^{99m}Tc would be an alkyleneamine oxime. For ^{99m}Tc, the preferred chelate is HMPAO, since the ^{99m}Tc-HMPAO readily crosses the membrane of the negatively charged liposomes.

Efficient internalisation of material into the liposomes of the present invention is dependent on the presence of entrapped modifying compound in the internal phase. In the case of a radiometal complex being the internalised material, the function of the entrapped modifying compound is to convert the radiometal complex, preferably comprising ¹¹¹In or ^{99m}Tc, into a more hydrophilic form that remains internalised within the liposome, resulting in a more efficient radiolabelling process. It is thus crucial that the modifying compound is retained inside the preformed liposomes such that the transmembrane passage of radiometal complex into the liposomes is not inhibited and that the radiometal complex is not converted into a hydrophilic form outside the liposomes, adversely affecting radiochemical purity (RCP).

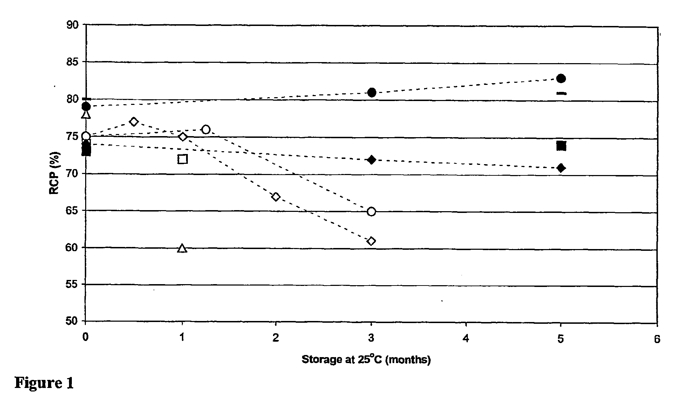

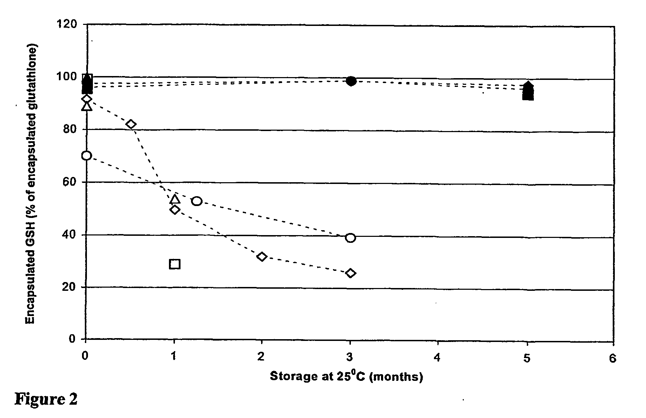

In Examples 1 and 3, Tables I and III show acceptable RCP of 70% for lyophilised DMPG- and DSPG-based liposomes containing 7.5-15 mole ratio cholesterol. In Example 2 it was demonstrated that the removal of buffer from the external phase gave good RCP maintenance and minimised the degree of glutathione leakage upon lyophilisation. In Example 4, Tables IV and V show that the kinetics of glutathione degradation and leakage of encapsulated glutathione are both slow under stressed conditions, concomitant with stable RCP values (within experimental errors). Figures 1 and 2 demonstrate the improved RCP and chemical stability of some selected lyophilised DMPG- and DSPG-based liposomes compared to liposome suspensions of the composition DSPC:DMPG:cholesterol:α-tocopherol (mole ratio 49:5:44:2).

A third aspect of the present invention provides a kit for the preparation of liposomes having internalised material. The term 'kit' is meant as a commercially acceptable embodiment of the invention designed for the facile preparation of liposomes having internalised material suitable for administration to humans. The kit of the invention comprises:
(i) a first compartment containing liposomes according to the first aspect of the invention, and
(ii) a second compartment containing the material according to the first aspect of the invention or a precursor thereof.

The components of the kit may be present in separate containers such as vials or alternatively as separate compartments of a single container such as a vial or a two-compartment syringe. Internalisation of material into the liposomes is achieved by first reconstituting the material, or a precursor thereof, with an appropriate solution followed by incubation of the solution with the liposomes. Where the vial contains a precursor of the material, the solution suitably contains the necessary reactants to produce the material, e.g. in the case of a radioactive compound, the appropriate radioiosotope. A preferred use of the liposomes produced therein is the administration to humans for the purposes of imaging infection, inflammation or tumours. In a most preferred mode of use, sterile lyophilised liposomes are incubated with sterile ^{99m}Tc-HMPAO to give ^{99m}Tc-labelled liposomes suitable for administration to humans.

The liposomes of the first compartment are prepared by:
(i) producing a population of liposomes of a first form in a suitable size distribution,
(ii) replacing the external phase of the liposomes with a medium different from the internal phase, and
(iii) processing the liposomes into a second form suitable for extended storage.
   Creating a population of liposomes with the size and size distribution appropriate for optimal biological performances may be achieved by a number of methods. For the preparation of commercial quantities, a preferred method is high pressure homogenisation. A single run or a number of runs may be carried out until a suitably-sized population of liposomes is produced.

The components of the internal phase may be encapsulated upon hydration of a homogenous mixture of the desired lipid components or during processing. A step is included in the processing to remove external modifying compound. Removal of external modifying compound may be carried out by centrifugation, diafiltration, dialysis or chromatography. Diafiltration is the most preferred method for replacement of the external phase.

In a preferred embodiment, the liposomes are processed into a form suitable for maintaining their physicochemical and biological properties during extended storage periods. The bilayer composition of the liposomes according to the invention renders them capable of being processed from a first suspension form into a second form suitable for extended storage without loss of entrapped modifying compound, alteration in liposome size or alteration of membrane fluidity. This second form may be a frozen or dehydrated version of the first form. Suitable methods to produce this second form are freezing, lyophilisation and vitrification. A preferred method is lyophilisation. In this second form, the liposomes are capable of retaining their original size as well as entrapped modifying compound in its original chemical form over extended storage periods. As demonstrated in Examples 1 and 3, the performance of DSPG-based and DMPG-based liposomes upon lyophilisation was only satisfactory when the cholesterol content was in the low range (Tables I and III). Minimal glutathione leakage, marginal alterations in liposome size and RCP were common features for both types of liposome formulations upon lyophilisation.

The liposomes of the present invention are preferably prepared in a form suitable for administration to humans for the imaging of infection, inflammation and tumours. It is therefore necessary to ensure that the liposomes (1) have appropriate physicochemical properties (e.g. size, size distribution and lipid bilayer fluidity), (2) have the desired internal and external aqueous phases, and (3) are sterile. A fourth aspect of the present invention is the use of the liposomes for imaging infection, inflammation and tumours in humans. The liposomes having internalised material of the invention are preferably used for the imaging of infection and/or inflammation in a human. Suitable infectious and inflammatory conditions include osteomyleitis, inflammatory bowel disease and appendicitis.

The biological performance of liposomes having internalised material is dependent on the stability of the preparation both *in vitro* and *in vivo* in relation to key physicochemical properties. For a liposome preparation it is crucial that the internalised material remains associated with the liposomes.

### Description of the figures

Figure 1 depicts the RCP stability of lyophilised DMPG- and DSPG-based liposomes (closed symbols) and liposome suspension (open symbols) upon storage at 25°C (see Example 4 for formulation details).
Figure 2 shows the chemical stability of encapsulated glutathione for lyophilised DMPG- and DSPG-based liposomes (closed symbols) and liposome suspension (open symbols) upon storage at 25°C. (see Example 4 for formulation details).

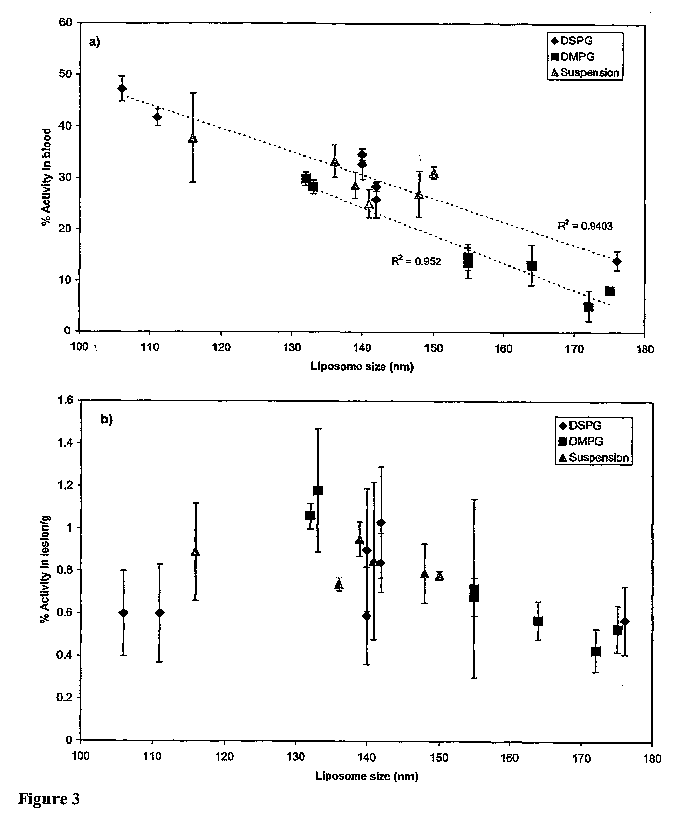

Figure 3 compares DSPG and DMPG with regard to percentage radioactivity in a) blood and b) lesion for reconstituted ^{99m}Tc-labelled lyophilised DSPC:DXPG:cholesterol (mole ratio: 49:5:X) liposomes (4 hrs pi, lipid dosage -2mg/kg bw). Results for liposome suspension are included. Data are given as mean ± SD (n=3). (see Example 6 for formulation details).
Figure 4 depicts; a) Predicted versus measured percentage radioactivity in blood for a partial least square (PLS-1) model using cholesterol content, DMPG/DSPG and liposome size as variables, b) Regression coefficients for the model.
Figure 5 shows the response surface of percentage radioactivity in blood as a function of liposome size and DMPG (0)/DSPG (1) for the PLS-model shown in Figure 4.

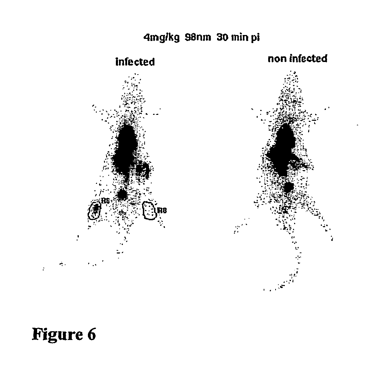

Figure 6 compares the gamma camera images post-injection of reconstituted ^{99m}Tc-labelled lyophilised DSPC:DSPG:cholesterol (mole ratio: 49:5:12.5) liposomes in infected and non-infected rat.

The invention is illustrated by the following non-limiting examples:

### Experimental examples

### Example 1 - Influence of cholesterol level on the performance of lyophilised glutathione containing liposomes.

A lipid mixture containing DSPC, DMPG (or DSPG) and cholesterol in a mole ratio of 49:5:X was prepared, where X varied between 0 and 44. Briefly, the lipids were dissolved in organic solvents and the resulting lipid mixture was dried under reduced pressure and sieved to produce a fine lipid powder.

A dispersion of lipids in a glutathione-containing 10 mM citrate-phosphate buffered 300 mM sucrose solution (pH 5) was subjected to the steps of high presssure homogenisation and/or extrusion. Removal of extraliposomal glutathione was achieved by diafiltration against 300 mM sucrose solution. The liposomes were then lyophilised.

Radiolabelling of the above-prepared liposomes was achieved by the diffusion of ^{99m}Tc-labelled HMPAO across the lipid bilayer. Briefly, ^{99m}Tc was obtained in the form of sodium pertechnetate solution by elution of a commercial ⁹⁹Mo/^{99m}Tc generator (Amertec II) and diluted to a concentration of 200 MBq/ml. The eluate was used within 2 hours after elution from the generator. One vial of HMPAO (Ceretec^{™}) was reconstituted with 5 ml of pertechnetate (200MBq/ml) and shaken for 10 seconds. The activity was between 900 MBq and 1100 MBq (±10%). After between 1 and 5 minutes 4ml of the radioactive solution was transferred to the freeze-dried liposomes and the mixture shaken for 10 seconds. The activity of the resulting solution was between 810 MBq and 990 MBq (±10%).

The physicochemical properties of lyophilised glutathione-containing DSPC:DMPG:cholesterol (mole ratio; 49:5:X) liposomes are summarised in Table I. Typically, the liposome size (Z-average; intensity-weighted mean diameter) was measured by photon correlation spectroscopy, the number concentration of large vesicles (1-30^{s}_{B}m) by the Coulter Counter technique, the RCP by instant thin layer chromatography, the extraliposomal and total content of glutathione by HPLC.

**Table I: Physicochemical properties of lyophilised DSPC:DMPG:cholesterol (mole ratio; 49:5:X) liposomes containing citrate-phosphate buffered (pH 5) and unbuffered sucrose solutions as internal and external phases, respectively.**

| **Batch No** | **cholesterol mole ratio (X)** | **Liposome size (nm) (change post-lyophilisation)** | **No. conc. large vesicles/ml (increase post lyophilisation)** | **glutathione leakage^{a} (%)** | **RCP (%) (change post lyophilisation)** |
|---|---|---|---|---|---|
| 1 | 0 | 157 (+43) | 2520x10⁶ (x1.6) | 27.2 | 30 (-8) |
| 2 | 5 | 140 (-2) | 2100x10⁶(x2.0) | <9.5 | 56 (-21) |
| 3 | 10 | 140 (0) | 2900x10⁶ (x0.7) | 5.1 | 75 (-1) |
| 4 | 7.5 | 175 (+4) | 2300x10⁶ (x1.6) | 3.2 | 76 (-4) |
| 5 | 10 | 160(+1) | 1100x10⁶ (x2.5) | 1.9 | 79 (-3) |
| 6 | 12.5 | 164 (+7) | 2780x10⁶ (x1.3) | 4.1 | 74 (0) |
| 7 | 15 | 117 (0) | 2260x10⁶ (x0.7) | 8.2 | 70 (-6) |
| 8 | 15 | 133 (+3) | 1300x10⁶ (x2.0) | 1.9 | 70 (-10) |
| 9 | 20 | 133 (+3) | 1680x10^{ö} (x1.8) | <3.4 | 56 (-20) |
| 10 | 44 | 122 (+10) | 1600x10⁶ (x19) | 7.6 | 54 (-26) |

| | | | | | |
|---|---|---|---|---|---|
| ^{a} reduced and oxidised glutathione | | | | | |

### Example 2 - Effect of removal of extraliposonlal buffer on the performance of lyophilised glutathione-containing liposomes.

Glutathione-containing liposomes of the composition DSPC:DMPG:cholesterol with a mole ratio 49:5:10 were prepared as described in Example 1. The internal phase contained a phosphate buffered 300 mM sucrose solution (pH 6). The external phase contained either a 300 mM sucrose solution or a phosphate buffered 300 mM sucrose solution (pH 6).

Table II summarises the effect of external buffer on the performance of glutathione-containing DSPC:DMPC:cholesterol (mole ratio; 49:5:10) liposomes during lyophilisation.

**Table II: Physicochemical properties of lyophilised DSPC:DMPG:cholesterol (mole ratio; 49:5:10) liposomes containing phosphate buffered sucrose solution (pH 6) as internal phase and either sucrose solution or phosphate buffered sucrose solution (pH 6) as external phase.**

| **Batch No** | **External Buffer (yes/no)** | **Liposome size (nm) (change post-lyophilisation)** | **glutathione leakage^{a} (%)** | **RCP (%) (change post- lyophilisation)** |
|---|---|---|---|---|
| 11 | No | 155 (-2) | 1.9 | 78 (-8) |
| 12 | Yes | 145 (-1) | 8.6 | 56 (-15) |

| | | | | |
|---|---|---|---|---|
| ^{a} reduced and oxidised glutathione | | | | |

### Example 3 - Use of DSPG in place of DMPG as negatively charged phosplaolipid

Glutathione-containing liposomes of the composition DSPC:DSPG:cholesterol with a mole ratio 49:5:X were prepared as outlined in Example 1, where X varied from 10 to 15. The internal phase contained a citrate-phosphate buffered 300 mM sucrose solution (pH 5) whilst the external phase was a 300 mM sucrose solution.

Table III summarises the physicochemical properties of lyophilised glutathione-containing DSPC:DSPG:cholesterol (mole ratio; 49:5:X) liposomes.

**Table III: Physicochemical properties of lyophilised DSPC:DSPG:cholesterol (mole ratio; 49:5:X)liposomes containing citrate-phosphate buffered (pH 5) and unbuffered sucrose solutions as internal and external phases, respectively.**

| **Batch No** | **Cholesterol mole ratio (x)** | **Liposome size (nm) (change post lyophilisation)** | **glutathione leakage^{a} (%)** | **RCP (%) (change post lyophilisation)** |
|---|---|---|---|---|
| 13 | 10 | 143 (+2) | 2.5 | 77 (-3) |
| 14 | 10 | 154(+12)" | 4.5 | 73 (-6) |
| 15 | 12.5 | 140(+3) | 2.8 | 74 (-4) |
| 16 | 12.5 | 120 (-3) | 1.2 | 80 (-3) |
| 17 | 15 | 130(+2) | 1.8 | 70-(-7) |

| | | | | |
|---|---|---|---|---|
| ^{a} reduced and oxidised glutathione, ^{b} erroneous size increase during lyophilisation (see Table V) | | | | |

### Example 4 - Storage of Ivophilised glutathione-containing liposomes over extended time periods.

The stability of various lyophilised liposome batches of the composition DSPC:DMPG:cholesterol and DSPC:DSPG:cholesterol (mole ratio; 49:5:X), where X varied from 10 to 15 (see Tables I-III), was monitored by storing vials in stability rooms with controlled temperature and humidity and protected from light. The various storage conditions used were 4°C/ambient humidity, 25°C/60% humidity, 30°C/60% humidity and 40°C/75% humidity. The following parameters were measured upon release, up to 5 months storage: extraliposomal and total glutathione content, liposome size, and RCP.

**Table IV: Stability data of selected lyophilised DSPC:DMPG:cholesterol (mole ratio; 49:5:X) liposomes.**

| **Batch No** | **Liposome size (nm)** | **Total glutathione^{a} (mg/mL)** | | **External glutathione (mg/mL)** | | **RCP (%)** |
|---|---|---|---|---|---|---|
| | | **glutathione** | **GSSG^{b}** | **glutathione** | **GSSG** | |
| 18 X=15 | | | | | | |
| Release | 143 | 1.22 | 0.11 | 0.09 | <0.04 | 68 |
| 12 w, 25°C | - | 1.25 | 0.10 | 0.07 | 0.02 | 69 |
| 5 m, 25°C | 137 | 1.13 | 0.13 | NA | NA | 66 |
| 19. X=10 | | | | | | |
| Release | 162 | 1.48 | 0.09 | 0.11 | <0.04 | 76 |
| 12 w, 25°C | - | 1.31 | 0.09 | 0.05 | 0.01 | 72 |
| 5 m, 25°C | 157 | 1.43 | <0.07 | NA | NA | 75 |
| 11. X=10 | | | | | | |
| Release | 155 | 1.46 | 0.16 | 0.01 | 0.03 | 78 |
| 2 m, 40°C | 149 | 1.30 | 0.15 | 0.04 | 0.02 | 80 |
| 5 m, 25°C | 153 | 1.32 | 0.17 | - | - | 82 |
| 6. X=12.5 | | | | | | |
| Release | 164 | 1.95 | 0.07 | 0.11 | 0.02 | 74 |
| 2 m, 25°C | 159 | 1.64 | 0.04 | 0.14 | 0.02 | 72 |
| 2 m, 40°C | 157 | 1.69 | 0.05 | 0.17 | 0.01 | 72 |
| 5 m, 25°C | 166 | 1.74 | 0.05 | 0.17 | 0.01 | 71 |

| | | | | | | |
|---|---|---|---|---|---|---|
| ^{a} reduced and oxidised glutathione, ^{b} oxidized glutathione | | | | | | |

**Table V: Stability data of selected lyophilised DSPC:DSPG:cholesterol (mole ratio; 49:5:X) liposomes.**

| **Batch No.** | **Liposome size (nm)** | **Total glutathione^{a} (mg/mL)** | | **External glutathione (mg/mL)** | | **RCP (%)** |
|---|---|---|---|---|---|---|
| | | **glutathione** | **GSSG^{b}** | **glutathione** | **GSSG** | |
| 13. X=10 | | | | | | |
| Release | 143 | 1.36 | 0.21 | 0.04 | 0.008 | 77 |
| 2 m, 30°C | 145 | 1.42 | 0.10 | 0.06 | 0.01 | 83 |
| 2 m, 40°C | 140 | 1.41 | 0.10 | 0.05 | 0.02 | 79 |
| 5 m, 25°C | 142 | 1.20 | 0.11 | 0.03 | 0.03 | 79 |
| 5 m, 40°C | 145 | - | - | - | - | 79 |
| 14. X=10 | | | | | | |
| Release | 154^{c} | 1.66 | 0.09 | 0.12 | 0.03 | 73 |
| 2 m, 30°C | 142 | 1.55 | 0.14 | - | - | 75 |
| 2 m, 40°C | 141 | 1.60 | 0.14 | - | - | 76 |
| 5 m, 25°C | 143 | 1.72 | 0.13 | 0.12 | 0.03 | 74 |
| 5 m, 40°C | 140 | 1.64 | 0.13 | 0.11 | 0.02 | 75 |
| 15. X=12.5 | | | | | | |
| Release | 140 | 1.60 | <0.010 | 0.06 | <0.008 | 74 |
| 2 m, 30°C | 138 | 1.47 | 0.08 | - | - | 73 |
| 2 m, 40°C | 139 | 1.56 | 0.08 | - | - | 74 |
| 5 m, 25°C | 139 | 1.55 | 0.08 | 0.06 | 0.009 | 74 |
| 5 m, 40_{°}C | 138 | 1.52 | 0.11 | 0.07 | 0.007 | 72 |
| 16. X=12.5 | | | | | | |
| Release | 122 | 1.45 | <0.1 | 0.03 | <0.008 | 80 |
| 2 m, 30°C | 124 | 1.39 | 0.08 | - | - | 84 |
| 2 m, 40°C | 123 | 1.36 | 0.1 | - | - | 81 |
| 5 m, 25°C | 121 | 1.40 | 0.08 | 0.021 | 0.007 | 81 |
| 5 m, 40°C | 122 | 1.31 | 0.11 | 0.034 | <0.007 | 79 |

| | | | | | | |
|---|---|---|---|---|---|---|
| ^{a} reduced and oxidised gluathione, ^{b} oxidised glutathione, large size probably due toinstrumental error. | | | | | | |

### Example 5 - Infected rat model.

200g (± 25g) male Wistar Rats were anaesthetised with Halothane. A focal abscess was created in the right thigh by inoculation of 0.1ml of 7 x 10⁸ cells/ml *S*. *Aureus* concentrate suspension into the gracilis muscle. The needle was removed post injection and the area wiped lightly with a cotton wool ball soaked in 70% ethanol solution. Animals were placed back into their cages and monitored carefully whilst recovering. Animals were studied within 48 hours of inoculation.

### Example 6-Biodistribution of lyophilised DSPC:DSPG:cholesterol and DSPC:DMPG: cholesterol liposomes in the infected rat model

Lyophilised DSPC:DMPG:cholesterol (mole ratio; 49:5:X) and DSPC:DSPG:cholesterol (mole ratio; 49:S:X) liposome batches, where X varied from 7.5 to 20, were investigated *in vivo* (see Tables I-III). Biodistribution studies were carried out in the *S. Aureus* infected rat model described in Example 5.

Figure III compares the % radioactivity in (a) blood, and (b) lesion 4 hours after intravenous injection of reconstituted ⁹⁹Tc-labelled lyophilised DSPG- and DMPG-based liposomes (lipid dosage, 2mg/kg body weight).

### Example 7 - Gamma camera image

Dynamic gamma camera images of rats were obtained following injection of liposomes of the invention (lipid dosage 4 mg/kg body weight).

Figure VI depicts a comparative image of an infected and a non-infected rat 30 minutes post injection of reconstituted ⁹⁹Tc-labelled lyophilised DSPC:DSPG:cholesterol (mole ratio; 49:5:12.5) liposomes.

## Claims

1. A liposome comprising:
i) a neutral phospholipid,
ii) a negatively charged phospholipid,
iii)a sterol,
having a modifying compound entrapped in the internal phase of said liposome;
wherein the modifying compound is a compound that is capable of irreversibly chemically modifying an intemalisable material within said liposome following diffusion of said material across the phospholipid bilayer of said liposome, such that the irreversibly chemically modified material remains internalised within said liposome, and;
wherein the neutral phospholipid is present at 60-90 mole percent of the total lipid content, the negatively charged phospholipid is present at 2-20 mole percent of the total lipid content and the sterol is present at 5-25 mole percent of the total lipid content.

2. The liposome of claim 1 wherein the modifying compound is a reductant.

3. The liposome of claims 1 and 2 wherein the modifying compound is chosen from ascorbic acid, cysteine and glutathione.

4. The liposome of claim 3 wherein the modifying compound is glutathione.

5. The liposome of claims 1-4 wherein the proportion of the modifying compound present in the internal phase of said liposome is in the range 95-100% of the modifying compound present in the internal and external phases of said liposome.

6. The liposome of claims 1-5 wherein said initernalisable material is chosen from a radioactive agent, a paramagnetic agent or a radioopaque agent.

7. The liposome of claims 1-6 wherein said intemalisable material is a metal chelate.

8. The liposome of claim 7 wherein the metal of said metal chelate is chosen from a radiometal suitable for SPECT or PET imaging, a paramagnetic metal suitable for MRI or a radioopaque metal suitable for x-ray imaging.

9. The liposomes of claims 7 and 8 wherein the metal of said metal chelate is selected from ^{99m}Tc ¹¹¹In, gadolinium, manganese or tungsten.

10. The liposome of claims 1-5 wherein said internalisable material is a prodrug.

11. The liposome of claims 1-10 wherein said sterol is present at 10-25 mole percent of the total lipid content.

12. The liposome of claims 1-11 wherein the fatty acyl chain length of said neutral phospholipid and that of said negatively charged phospholipid is in the range 14 to 20 carbon atoms.

13. The liposome of claims 1-12 wherein the fatty acyl chains of said neutral phospholipid and of said negatively charged phospholipid are of equal length.

14. The liposome of claims 1-13 wherein the fatty acyl chains of said neutral phospholipid and said negatively charged phospholipid are chosen from myristic acid, palmitic acid, stearic acid, oleic acid, linoleic acid and arachidonic acid.

15. The liposome of claims 1-14 wherein said neutral phospholipid is a phosphatidylcholine compound.

16. The liposome of claims 1-15 wherein said neutral phospholipid is distearoylphosphatidylcholine (DSPC) or dimyristoylphosphatidylcholine (DMPC).

17. The liposome of claims 1-16 wherein said negatively charged phospholipid is a phosphatidylglycerol compound, a phosphatidylserine compound, or a phosphatidylinositol compound.

18. The liposome of claims 1-17 wherein said negatively charged phospholipid is distearoylphosphatidylglycerol (DSPG) or dimyristoylphosphatidylglycerol (DMPG).

19. The liposome of claims 1-18 wherein the mean liposome diameter is in the range 50 and 400nm.

20. The liposome of claims 1-19 wherein the mean liposome diameter is in the range 80 and 140nm.

21. A liposome having an internalised material which comprises:
i) the liposome of claims 1-20,
ii) the irreversibly chemically modified material of claims 1 to 10.

22. The liposome of claim 21 wherein the internalised material is a SPECT imaging agent, an MRI contrast agent or an x-ray contrast agent.

23. The liposome of claim 21 wherein the internalised material is a drug.

24. A kit for the preparation of the liposome of claims 21-22 which comprises:
i) a first compartment containing the liposome of claims 1-9 and claims 11-20, and
ii) a second compartment containing; the intemalisable material of claims 6 to 9 or a precursor thereof.

25. A kit for the preparation of the liposome of claim 23 which comprises:
i) a first compartment containing the liposome of claims 1-5 and claims 10-20, and
ii) a second compartment containing the intemalisable material of claim 10 or a precursor thereof.

26. The kit of claims 24 or 25 wherein the liposome of said first compartment and the material or pre:cursor thereof of said second compartment are lyophilised.

27. The kit of claims 24 to 26 wherein said first compartment further comprises a cryoprotectant.

28. The kit of claim 27 wherein the cryoprotectant is sucrose.

29. The liposome of claim 22 for use in imaging infection, inflammation or tumour in a human.

30. The liposome of claim 29 for use in imaging osteomyleitis, inflammatory bowel disease and appendicitis.

## Patentansprüche

1. Liposom, umfassend:
i) ein neutrales Phospholipid,
ii) ein negativ geladenes Phospholipid,
iii) ein Sterol,
mit einer modifizierenden Verbindung, die in der inneren Phase des Liposoms gefangen ist;
wobei die modifizierende Verbindung eine Verbindung ist, die in der Lage ist, ein internalisierbares Material innerhalb des Liposoms irreversibel chemisch zu modifizieren im Anschluss an eine Diffusion des Materials durch die Phospholipiddoppelschicht des Liposoms, so dass das irreversibel chemisch modifizierte Material innerhalb des Liposoms internalisiert bleibt, und;
wobei das neutrale Phospholipid vorhanden ist mit 60-90 Molprozent des Gesamtlipidgehalts, das negativ geladene Phospholipid vorhanden ist mit 2-20 Molprozent des Gesamtlipidgehalts und das Sterol vorhanden ist mit 5-25 Molprozent des Gesamtlipidgehalts.

2. Liposom nach Anspruch 1, wobei die modifizierende Verbindung ein Reduktionsmittel ist.

3. Liposom nach Ansprüchen 1 und 2, wobei die modifizierende Verbindung aus Ascorbinsäure, Cystein und Glutathion ausgewählt ist.

4. Liposom nach Anspruch 3, wobei die modifizierende Verbindung Glutathion ist.

5. Liposom nach Ansprüchen 1-4, wobei der Anteil der modifizierenden Verbindung, der in der inneren Phase des Liposoms vorhanden ist, im Bereich von 95-100% der modifizierenden Verbindung liegt, die in der inneren und äußeren Phase des Liposoms vorhanden ist.

6. Liposom nach Ansprüchen 1-5, wobei das internalisierbare Material aus einem radioaktiven Stoff, einem paramagnetischen Stoff oder einem radioopaken Stoff ausgewählt ist.

7. Liposom nach Ansprüchen 1-6, wobei das internalisierbare Material ein Metallchelat ist.

8. Liposom nach Anspruch 7, wobei das Metall des Metallchelats ausgewählt ist aus einem Radiometall, das sich für SPECT- oder PET-Bildgebung eignet, einem paramagnetischen Metall, das sich für MRI eignet, oder einem radioopaken Metall, das sich für Röntgenbildgebung eignet.

9. Liposom nach Ansprüchen 7 und 8, wobei das Metall des Metallchelats aus ^{99m}Tc, ¹¹¹In, Gadolinium, Mangan oder Wolfram ausgewählt ist.

10. Liposom nach Ansprüchen 1-5, wobei das internalisierbare Material ein Prodrug ist.

11. Liposom nach Ansprüchen 1-10, wobei das Sterol vorhanden ist mit 10-25 Molprozent des Gesamtlipidgehalts.

12. Liposom nach Ansprüchen 1-11, wobei die Fettsäurekettenlänge des neutralen Phospholipids und jene des negativ geladenen Phospholipids im Bereich von 14 bis 20 Kohlenstoffatomen liegt.

13. Liposom nach Ansprüchen 1-12, wobei die Fettsäureketten des neutralen Phospholipids und des negativ geladenen Phospholipids gleich lang sind.

14. Liposom nach Ansprüchen 1-13, wobei die Fettsäureketten des neutralen Phospholipids und des negativ geladenen Phospholipids ausgewählt sind aus Myristinsäure, Palmitinsäure, Stearinsäure, Ölsäure, Linolsäure und Arachidonsäure.

15. Liposom nach Ansprüchen 1-14, wobei das neutrale Phospholipid eine Phosphatidylcholinverbindung ist.

16. Liposom nach Ansprüchen 1-15, wobei das neutrale Phospholipid Distearoylphosphatidylcholin (DSPC) oder Dimyristoylphosphatidylcholin (DMPC) ist.

17. Liposom nach Ansprüchen 1-16, wobei das negativ geladene Phospholipid eine Phosphatidylglycerolverbindung, eine Phosphatidylserinverbindung oder eine Phosphatidylinositolverbindung ist.

18. Liposom nach Ansprüchen 1-17, wobei das negativ geladene Phospholipid Distearoylphosphatidylglycerol (DSPG) oder Dimyristoylphosphatidylglycerol (DMPG) ist.

19. Liposom nach Ansprüchen 1-18, wobei der mittlere Liposomdurchmesser im Bereich von 50 nm und 400 nm ist.

20. Liposom nach Ansprüchen 1-19, wobei der mittlere Liposomdurchmesser im Bereich von 80 und 140 nm ist.

21. Liposom mit einem internalisierten Material, das umfasst:
i) das Liposom nach Ansprüchen 1-20,
ii) das irreversibel chemisch modifizierte Material nach Ansprüchen 1 bis 10.

22. Liposom nach Anspruch 21, wobei das internalisierte Material ein SPECT-Bildgebungsmittel, ein MRI-Kontrastmittel oder ein Röntgenkontrastmittel ist.

23. Liposom nach Anspruch 21, wobei das internalisierte Material ein Wirkstoff ist.

24. Kit zur Herstellung des Liposoms nach Ansprüchen 21-22, das umfasst:
i) ein erstes Kompartiment, welches das Liposom nach den Ansprüchen 1-9 und den Ansprüchen 11-20 enthält, und
ii) ein zweites Kompartiment, welches das internalisierbare Material nach den Ansprüchen 6 bis 9 oder einen Vorläufer davon enthält.

25. Kit zur Herstellung des Liposoms nach Anspruch 23, das umfasst:
i) ein erstes Kompartiment, welches das Liposom nach den Ansprüchen 1-5 und den Ansprüchen 10-20 enthält, und
ii) ein zweites Kompartiment, welches das internalisierbare Material nach Anspruch 10 oder einen Vorläufer davon enthält.

26. Kit nach Anspruch 24 oder 25, wobei das Liposom des ersten Kompartiments und das Material oder dessen Vorläufer des zweiten Kompartiments lyophilisiert sind.

27. Kit nach Ansprüchen 24 bis 26, wobei das erste Kompartiment weiterhin ein Kryoprotektivum umfasst.

28. Kit nach Anspruch 27, wobei das Kryoprotektivum Saccharose ist.

29. Liposom nach Anspruch 22 zur Verwendung bei der Bildgebung von Infektion, Entzündung oder einem Tumor beim Menschen.

30. Liposom nach Anspruch 29 zur Verwendung bei der Bildgebung von Osteomyelitis, chronisch entzündlicher Darmerkrankung und Appendizitis.

## Revendications

1. Liposome comprenant :
i) un phospholipide neutre,
ii) un phospholipide chargé négativement,
iii) un stérol,
comportant un composé de modification piégé dans la phase interne dudit liposome ;
dans lequel le composé de modification est un composé qui peut modifier chimiquement de manière irréversible un matériau pouvant être intégré à l'intérieur dudit liposome suite à la diffusion dudit matériau à travers la bicouche de phospholipide dudit liposome, de sorte que le matériau modifié chimiquement de manière irréversible reste intégré dans ledit liposome, et ;
dans lequel le phospholipide neutre est présent à une teneur de 60 à 90 moles pour-cent de la teneur en lipide totale, le phospholipide chargé négativement est présent à une teneur de 2 à 20 moles pour-cent de la teneur en lipide totale et le stérol est présent à une teneur de 5 à 25 moles pour-cent de la teneur en lipide totale.

2. Liposome selon la revendication 1, dans lequel le composé de modification est un réducteur.

3. Liposome selon les revendications 1 et 2, dans lequel le composé de modification est choisi à partir d'acide ascorbique, de cystéine et de glutathione.

4. Liposome selon la revendication 3, dans lequel le composé de modification est le glutathione.

5. Liposome selon les revendications 1 à 4, dans lequel la proportion du composé de modification présent dans la phase interne dudit liposome est dans la plage de 95 à 100 % du composé de modification présent dans les phases interne et externe dudit liposome.

6. Liposome selon les revendications 1 à 5, dans lequel ledit matériau pouvant être intégré est choisi à partir d'un agent radioactif, d'un agent paramagnétique ou d'un agent radio-opaque.

7. Liposome selon les revendications 1 à 6, dans lequel ledit matériau pouvant être intégré est un chelate métallique.

8. Liposome selon la revendication 7, dans lequel le métal dudit chélate métallique est choisi à partir d'un radiométal approprié pour l'imagerie SPECT ou PET, un métal paramagnétique approprié pour l'IRM ou un métal radio-opaque approprié pour la radiographie.

9. Liposome selon les revendications 7 et 8, dans lequel le métal dudit chélate métallique est sélectionné à partir du ^{99m}Tc, du ¹¹¹In, du gadolinium, du manganèse ou du tungstène.

10. Liposome selon les revendications 1 à 5, dans lequel ledit matériau pouvant être intégré est une prodrogue.

11. Liposome selon les revendications 1 à 10, dans lequel ledit stérol est présent à une teneur de 10 à 25 moles pour-cent de la teneur en lipide totale.

12. Liposome selon les revendications 1 à 11, dans lequel la longueur de la chaîne d'acyle gras dudit phospholipide neutre et celle du phospholipide chargé négativement sont dans la plage de 14 à 20 atomes de carbone.

13. Liposome selon les revendications 1 à 12, dans lequel les chaînes d'acyle gras dudit phospholipide neutre et celles du phospholipide chargé négativement sont de longueur égale.

14. Liposome selon les revendications 1 à 13; dans lequel les chaînes d'acyle gras dudit phospholipide neutre et dudit phospholipide chargé négativement sont choisies de l'acide myristique, l'acide palmitique, l'acide stéarique, l'acide oléique, l'acide linoléïque et de l'acide arachidonique.

15. Liposome selon les revendications 1 à 14, dans lequel ledit phospholipide neutre est un composé de phosphatidylcholine.

16. Liposome selon les revendications 1 à 15, dans lequel ledit phospholipide neutre est du distéaroylphosphatidylcholine (DSPC) ou du dimyristoylphosphatidylcholine (DMPC).

17. Liposome selon les revendications 1 à 16, dans lequel le phospholipide chargé négativement est un composé de phosphatidylglycérol, un composé de phosphatidylsérine, ou composé de phosphatidylinositol.

18. Liposome selon les revendications 1 à 17, dans lequel le phospholipide chargé négativement est du distéaroylphosphatidylglycérol (DSPG) ou du dimyristoylphosphatidylglycérol (DMPG).

19. Liposome selon les revendications 1 à 18, dans lequel le diamètre moyen de liposome est compris dans la plage de 50 à 400 nm.

20. Liposome selon les revendications 1 à 19, dans lequel le diamètre moyen de liposome est compris dans la plage de 80 à 140 nm,

21. Liposome comportant un matériau intégré qui comprend:
i) le liposome selon les revendications 1 à 20,
ii) le matériau modifié chimiquement de manière irréversible selon les revendications 1 à 10.

22. Liposome selon la revendication 21, dans lequel le matériau intégré est un agent d'imagerie SPECT, un agent de contraste d'IRM ou un agent de contraste de radiographie.

23. Liposome selon la revendication 21, dans lequel le matériau intégré est un médicament.

24. Kit de préparation du liposome selon les revendications 21 et 22 qui comprend :
i) un premier compartiment contenant un liposome selon les revendications 1 à 9 et les revendications 11 à 20, et
ii) un second compartiment contenant le matériau pouvant être intégré selon les revendications 6 à 9 ou un précurseur de celui-ci.

25. Kit de préparation du liposome selon la revendication 23 qui comprend :
i) un premier compartiment contenant le liposome selon les revendications 1 à 5 et les revendications 10 à 20, et
ii) un second compartiment contenant le matériau pouvant être intégré selon la revendication 10 ou un précurseur de celui-ci.

26. Kit selon les revendications 24 ou 25, dans lequel le liposome dudit premier compartiment et le matériau ou son précurseur dudit second compartiment sont lyophilisés.

27. Kit selon les revendications 24 ou 26, dans lequel ledit premier compartiment comprend, en outre, un agent cryoprotecteur.

28. Kit selon la revendication 27, dans lequel l'agent cryoprotecteur est du sucrose.

29. Liposome selon la revendication 22, destiné à être utilisé en imagerie d'infection, d'inflammation ou de tumeur sur un humain.

30. Liposome selon la revendication 29, destiné à être utilisé en imagerie de l'ostéomyélite, les maladies inflammatoires de l'intestin et appendicite.
